# EUROPEAN PATENT APPLICATION

(11) **EP 1 479 386 A1**
(43) Date of publication of application: **24.11.2004**
(21) Application number: 03009921.2
(22) Date of filing: 30.04.2003
(51) Int. Cl.: A61K 31/47, A61K 31/4725, A61K 31/496, A61P 17/00

(54) **Use of isoquinoline-derivatives for treating and preventing pigmentary disorders**

(71) Applicant: Switch Biotech Aktiengesellschaft, 82061 Neuried (DE)
(72) Inventor: Bell, Stefan, 81379 München (DE); Ewald, Dagmar, 85402 Kranzberg (DE); Fritz, Stephanie, 81541 München (DE); Goppelt, Andreas, 80636 München (DE)
(74) Representative: Krauss, Jan

(57) **Abstract**

The invention relates to the use of isoquinoline-derivatives for treating and/or preventing pigmentary disorders.

## Description

The invention relates to the use of isoquinoline-derivatives for treating and/or preventing pigmentary disorders.

### State of the Art

In the skin or hair, melanocytes are the sole source of the pigment melanin. Melanin is synthesized within the melanocytes and later transferred to the surrounding keratinocytes. The colour of the skin is determined to a large extent by the amount and type of melanin within the epidermis. In general dysfunction of the melanocytes or the loss of the melanocytes itself leads to loss of pigmentation. The mechanisms for the destruction of melanocytes are likely to be multiple and complex, possibly a composite of several normal processes influencing melanocyte function, proliferation and/or survival. Also the pathomechanism of hyperpigmentary disorders is largely unclear.

Vitiligo, for example is a pigmentation disorder afflicting up to 2% of the worldwide population. It is a specific type of leukoderma manifested characteristically by depigmentation of the epidermis, best described as an acquired, progressive disorder that selectively destroys some or all melanocytes. The vitiligo disease is characterized by milky white macules on the skin, either due to missing melanin pigment or to complete absence of melanocytes in the dermo-epidermal junction of vitiligo areas. Vitiligo tends to be progressive throughout the life of affected individuals. Other disorders of hypopigmentation that are caused by a defect in melanin production or transfer include the Chediak-Higashi syndrome, Hermansky-Pudlak syndrome, the Waardenburg syndromes I-IV, the Angelman and Prader-Willi syndrome. Albinism instead is characterized by genetic defects that impede the synthesis of melanin. Piebaldism is characterized by the absence of melanin at birth due to a deficiency of melanocytes. During embryogenesis melanocytes fail to complete their migration from the neural crest to the epidermis. In vitiligo some melanocytes can be found in epidermis of early lesions that are only partially depigmented. In late lesions that were totally depigmented, there is complete absence of melanocytes. Also, there is a lack of knowledge about the pathophysiology and mechanisms underlying most pigmentary disorders.

At the moment there are still several hypothesis to explain the possible causes of vitiligo:
1) Autoimmune disease: Specific autoantibodies to melanocyte cell surface antigens are present in the circulation of most patients with vitiligo. These antibodies are unusual in persons with nonpigmentary skin diseases. Vitiligo antibodies are shown to have the functional capacity to kill pigment cells in vitro and can do so by two different mechanisms: complement-dependent cytotoxicity and antibody-dependent cellular cytotoxicity.
2) Self-destruction of melanocytes as a consequence of aberrant melanin biosynthesis: The autocytotoxic hypothesis is based on the observation that phenol and some of its derivatives are capable of killing pigment cells. Tyrosine, the substrate of the tyrosinase is itself a phenol derivative, is oxidized into melanin via a complex series of oxidative reactions. Some intermediates are capable of forming radicals. It is thought that melanin synthesis is confined within the melanosome to prevent these melanin precursors from diffusing into the cell where they might disrupt essential metabolic pathways.
3) Overproduction of neurotransmitters leading to death of melanocytes: Melanocytes are neural crest derived cells. A dysfunction of nervous function might be involved in the pathogenesis of vitiligo as shown by an altered balance of neuropeptides in vitiliginous skin. Neuropeptides are able to induce melanocyte dendricity and participate in the regulation of cell substrate adhesion, cell motility and shape. Neuropeptides may also regulate melanin synthesis or affect melanosomal transfer to surrounding keratinocytes.
4) More recently, also viral infections as well as oxidative stress and hormonal causes are discussed to be involved in the disease formation.
5) Although the pathogenesis of vitiligo is still not known, there is a genetic predisposition, demonstrated by the fact, that 40% of vitiligo patients have a positive family history for this disease.

Currently, no satisfying treatments exist for pigmentary disorders. For example, at present, there is no specific therapy for Vitiligo available without side effects and no innovative therapeutic programs are under development. No single therapy predictably produces good results in all patients and the responses are highly variable: systemic photochemotherapy (PUVA) gives satisfactory results only in some early disease states, however, treatment is time-consuming and has a high risk of developing cancer after prolonged treatment. Other therapies comprise systemic steroids,e.g. prednisone, hydrocortisone or triamcinolone, which however are also not suitable for prolonged treatment. In some cases, transplantation of skin has given positive results. In extreme cases, where the depigmented area has become very large, total chemical depigmentation of the skin is performed to achieve a homogeneous coloring of the skin.

Similarly, no specific and satisfactory treatment exists for Pityriasis alba, a common hypopigmented dermatitis that occurs primarily in school-aged children. Usually this disorder is left untreated as treatments with corticosteroids or retinoic acid or PUVA treatment are not very effecient.

Some pigmentary disorders, like vitiligo, have only skin manifestations limited to the pigmentation alterations. However, these disorders nevertheless pose severe psychological problems to the patients, as the sharp borders of depigmented areas are readily apparent to other persons, especially when occuring in the face. Vitiligo can be disfiguring and stigmatising, thereby causing significant psychological problems due to reduced social acceptance. Also, vitiligo usually persists for the whole life.

There is therefore a need for a specific treatment of pigmentary disorders, of hair and skin, especially of hypopigmentary disorders, preferably vitiligo.

### Disclosure of the present invention

Surprisingly it was found that isoquinoline-derivatives are useful in the treatment and prevention of pigmentary disorders.

### The present invention therefore relates to the

Use of a compound according to formula (I): 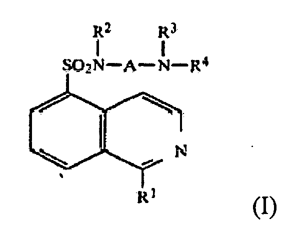 or an isomer thereof, or a pharmaceutically acceptable salt thereof, for the manufacture of a medicament for treating and/or preventing pigmentary disorders, preferably hypopigmentary disorders, especially vitiligo,
wherein
R¹ is a hydrogen, a chlorine or a hydroxy, and
when R' is a hydrogen:
A is an alkylene having 2 to 6 carbon atoms, which optionally has an alkyl having 1 to 10 carbon atoms, aryl or aralkyl as a substituent;
R² is a hydrogen, or a linear or branched alkyl having 1 to 6 carbon atoms, an aryl or an aralkyl;
R³ is a hydrogen, or a linear or branched alkyl having 1 to 6 carbon atoms, an aryl or an aralkyl;
R⁴ is a hydrogen, a linear or branched alkyl having 1 to 6 carbon atoms, an aryl or an aralkyl, or a benzoyl, a cinnamyl, a cinnamoyl, a furoyl or a group of the following formula 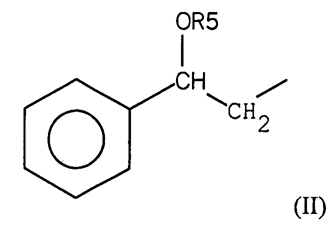 wherein R⁵ is a linear or branched alkyl having 1 to 6 carbon atoms or a group of the following formula 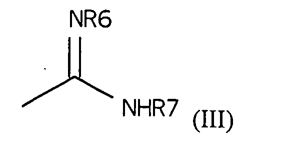 wherein R⁶ and R⁷ are hydrogen or directly bonded to form alkylene having 2 to 4 carbon atoms; or
   R² and R³ are directly bonded to each other to form an alkylene having 4 or less carbon atoms, which is optionally substituted by an alkyl having 1 to 10 carbon atoms, phenyl or benzyl, or
   R³ and R⁴ directly or in combination via oxygen atom form a heterocycle together with the adjacent nitrogen atom, and
when R¹ is a chlorine or a hydroxy:
A is an alkylene having 2 to 6 carbon atoms, which is optionally substituted by an alkyl having 1 to 6 carbon atoms,
R² and R³ are each independently hydrogen, a linear or branched alkyl having 1 to 6 carbon atoms, or R₂ and R₃ are directly bonded to each other to form ethylene or trimethylene, which ethylene or trimethylene is optionally substituted by an alkyl having 1 to 6 carbon atoms; and
R⁴ is hydrogen, a linear or branched alkyl having 1 to 6 carbon atoms or an amidino.

In a preferred embodiment, the compound is selected from the group consisting of:
1-(5-isoquinolinesulfonyl)homopiperazine
1-(5-isoquinolinesulfonyl)-2-methylhomopiperazine
1-(5-isoquinolinesulfonyl)-3-methylhomopiperazine
1-(5-isoquinolinesulfonyl)-6-methylhomopiperazine
1-(5-isoquinolinesulfonyl)-2,3-dimethylhomopiperazine
1-(5-isoquinolinesulfonyl)-3,3-dimethylhomopiperazine
1-(5-isoquinolinesulfonyl)-3-ethylhomopiperazine
1-(5-isoquinolinesulfonyl)-3-propylhomopiperazine
1-(5-isoquinolinesulfonyl)-3-isobutylhomopiperazine
1-(5-isoquinolinesulfonyl)-3-phenylhomopiperazine
1-(5-isoquinolinesulfonyl)-3-benzylhomopiperazine
1-(5-isoquinolinesulfonyl)-6-ethylhomopiperazine
1-(5-isoquinolinesulfonyl)-6-propylhomopiperazine
1-(5-isoquinolinesulfonyl)-6-butylhomopiperazine
1-(5-isoquinolinesulfonyl)-6-pentylhomopiperazine
1-(5-isoquinolinesulfonyl)-6-hexylhomopiperazine
1-(5-isoquinolinesulfonyl)-6-phenylhomopiperazine
1-(5-isoquinolinesulfonyl)-6-benzylhomopiperazine
1-(5-isoquinolinesulfonyl)-4-methylhomopiperazine
1-(5-isoquinolinesulfonyl)-4-ethylhomopiperazine
1-(5-isoquinolinesulfonyl)-4-propylhomopiperazine
1-(5-isoquinolinesulfonyl)-4-butylhomopiperazine
1-(5-isoquinolinesulfonyl)-4-hexylhomopiperazine
N-(2-aminoethyl)-1-chloro-5-isoquinolinesulfonamide
N-(4-aminoethyl)-1-chloro-5-isoquinolinesulfonamide
N-(2-amino-1-methylethyl)-1-chloro-5-isoquinolinesulfonamide
N-(2-amino-1-methylpentyl)-1-chloro-5-isoquinoline
N-(3-amino-2-methylbutyl)- 1-chloro-5-isoquinolinesulfonamide
N-(3-di-n-butylaminopropyl)-1-chloro-5-isoquinolinesulfonamide
N-( N-cyclohexyl-N-methylaminoethyl)-1-chloro-5-isoquinolinesulfonamide
N-(2-guanidinoethyl)-1-chloro-5-isoquinolinesulfonamide
N-(2-guanidinobutyl)-5-chloro-5-isoquinolinesulfonamide
N-(2-guanidino-1-methylethyl)-1-chloro-5-isoquinolinesulfonamide
N-(2-guanidinomethylpentyl)-1-chloro-5-isoquinolinesulfonamide
N-(2-guanidino-3-methylbutyl)-1-chloro-5-isoquinolinesulfonamide
N-(3-guanidino-2-methylpropyl)-1-chloro-5-isoquinolinesulfonamide
N-(4-guanidino-3-methylbutyl)-1-chloro-5-isoquinolinesulfonamide
2-methyl-4-( 1-chloro-5-isoquinolinesulfonyl)piperazine
2-ethyl-4-(1-chloro-5-isoquinolinesulfonyl)piperazine
2-isobutyl-4-(1-chloro-5-isoquinolinesulfonyl)piperazine
2,5-dimethyl-4-(1-chloro-5-isoquinolinesulfonyl)piperazine
1-methyl-4-(1-chloro-5-isoquinolinesulfonyl)piperazine
1-amidino-4-(1-chloro-5-isoquinolinesulfonyl)piperazine
1-amidino4-(1-chloro-5-isoquinolinesulfonyl)homopiperazine
1-amidino-3-methyl-4-(1-chloro-5-isoquinolinesulfonyl)piperazine
1-amidino-2,5-dimethyl-4-(1-chloro-5-isoquinolinesulfonyl)piperazine
N-(2-aminoethyl)-1-hydroxy-5-isoqmnolinesulfonamide
N-(4-aminobutyl)-1-hydroxy-5-isoquinolinesulfonamide
N-(2-amino-1-methylethyl)-1-hydroxy-5-isoquinolinesulfonamide
N-(2-amino-1-methylheptyl)-1-hydroxy-5-isoquinolinesulfonamide
N-(3-amino-2-methylbutyl)-1-hydroxy-5-isoquinolinesulfonamide
N-[3-(N,N-dibutylamino)propyl]-1-hydroxy-5-isoquinolinesulfonamide
N-[2-(N-cyclohexyl-N-methylamino)ethyl]-1-hydroxy-5-isoquinolinesulfonamide
N-(2-guanidinoethyl)-1-hydroxy-5-isoquinolinesulfonamide
N-(4-guanidinobutyl)-1-hydroxy-5-isoquinolinesulfonamide
N-(2-guanidino-1)-methylethyl)-1-hydroxy-5-isoquinolinesulfonamide
N-(1-guanidinomethylpentyl)-1-hydroxy-5-isoquinolinesulfonamide
N-(2-guanidino-3-methylbutyl)-1-hydroxy-5-isoquinolinesulfonamide
N-(3-guanidino-2-methylpropyl)-1-hydroxy-5-isoquinolinesulfonamide
N-(4-guanidino-3-methylbutyl)-1-hydroxy-5-isoquinolinesulfonamide
2-methyl-4-( 1-hydroxy-5-isoquinolinesulfonyl)piperazine
2-ethyl-4-(1-hydroxy-5-isoquinolinesulfonyl)piperazine
2-isobutyl-4-(1-hydroxy-5-isoquinolinesulfonyl)piperazine
2,5-dimethyl-4-(1-hydroxy-5-isoquinolinesulfonyl)piperazine
1-methyl-4-(1-hydroxy-5-isoquinolinesulfonyl)piperazine
1-amidino-4-(1-hydroxy-5-isoquinolinesulfonyl)piperazine
1-amidino-4-(1-hydroxy-5-isoquinolinesulfonyl)homopiperazine
1-amidino-3-methyl-4-(1-hydroxy-5 -isoquinolinesulfonyl)piperazine
1-amidino-2,5-dimethyl-4-(1-hydroxy-5-isoquinolinesulfonyl)piperazine
N-(2-methylaminoethyl)-1-chloro-5-isoquinolinesulfonamide
N-(2-ethylaminoethyl)-1-chloro-5-isoquinolinesulfonamide
N-(2-propylaminoethyl)-1-chloro-5-isoquinolinesulfonamide
N-(2-butylaminoethyl)-1-chloro-5-isoquinolinesulfonamide
N-(2-hexylaminoethyl)-1-chloro-5-isoquinolinesulfonamide
1-(1-chloro-5-isoquinolinesulfonyl)piperazine
1-(1-chloro-5-isoquinolinesulfonyl)homopiperazine
N-(2-methylaminoethyl)-1-hydroxy-5-isoquinolinesulfonamide
N-(2-ethylaminoethyl)-1-hydroxy-5-isoquinolinesulfonamide
N-(2-propylaminoethyt)-1-hydroxy-5-isoquinolinesulfonamide
N-(2-butylaminoethyl)-1-hydroxy-5-isoquinolinesulfonamide
N-(2-hexylaminoethyl)-1-hydroxy-5-isoquinolinesulfonamide
1-(1-hydroxy-5-isoquinolinesulfonyl)piperazine
1-(1-hydroxy-5-isoquinolinesulfonyl)homopiperazine
1-(5-isoquinolinesulfonyl)-4-methylpiperazine
1-(5-isoquinolinesulfonyl)-4-n-hexylpiperazine
1-(5-isoquinolinesulfonyl)-4-cinnamylpiperazine
1-(5-isoquinolinesulfonyl)piperazine
N-(4-aminobutyl)-5-isoquinolinesulfonamide
N-(3-di-n-butylaminopropyl)-5-isoquinolinesulfonamide
1-(5-isoquinolinesulfonyl)-3-methylpiperazine
1-(5-isoquinolinesulfonyl)-3-isobutylpiperazine
1-(5-isoquinolinesulfonyl)-2,5-dimethylpiperazine
N-(3-guanidino-2-phenylpropyl)-5-isoquinolinesulfonamide
N-(6-guanidino-1-methylheptyl)-5-isoquinolinesulfonamide
2-[2-(5-isoquinolinesulfonamide)ethylamino]-2-imidazoline
2-amidino-1-(5-isoquinolinesulfonyl)piperazine
4-amidino-2,5-dimethyl-1-(5-isoquinolinesulfonyl)piperazine
4-amidino-1-(5-isoquinolinesulfonyl)homopiperazine
4-(N1,N2-dimethylamidino)-1-(5-isoquinolinesulfonyl)piperazine
4-amidino-3-butyl-1-(5-isoquinolinesulfonyl)piperazine
4-hexyl-1-(5-isoquinolinesulfonyl)ethylenediamine
N-(4-guanidinobutyl)-5-isoquinolinesulfonamide
N-(2-guanidinoethyl)-5-isoquinolinesulfonamide

In an even more preferred embodiment, the compound is 1-(5-isoquinolinesulfonyl)homopiperazine.

Methods of production of the compounds according to the present invention are well known to someone skilled in the art, and are described e.g. in EP 0 187 371, the contents of which is hereby incorporated by reference.

### Pigmentary disorders

Pigmentary disorders according to the present invention are non-malignant disorders which are characterized by an altered pigmentation of the affected tissue compared to healthy individuals.

Tissues affected by the pigmentary disorders comprise for example skin and hair, preferably skin. In a preferred embodiment , the pigmentary disorder is a hypopigmentary disorder; i.e. a disorder characterized by a diminished pigmentation or a loss of pigmentation compared to healthy individuals. Examples of such disorders are, but not limited to, albinism, vitiligo, piebaldism, Pityariasis alba, Hypomelanoses, Leukodermas, hypopigmentation occuring e.g. after externally induced peels, e.g. chemical peels with phenol, or laser or cryo-surgery of the skin, Chediak-Higashi syndrome, Hermansky-Pudlak syndrome, the Angelman and Prader-Willi syndrome. An especially preferred hypopigmentary disorder of the present invention is vitiligo.

In another preferred embodiment, the pigmentary disorder is characterized by hyperpigmentation of a tissue, preferably skin. Preferably, the hyperpigmentation is only partial. In the case of skin being affected by the disorder, compounds of the present invention may be used for obtaining a more regular pigmentation pattern. Examples for such disorders characterized by partial hyperpigmentation of a tissue, preferably skin, are but not limited to Lentigo and Ephelides.

Treatment according to the present invention relates to the complete or partial healing of the disorder as well as to the stop or slowing-down of the progression of a disorder. Also, the compounds of the present invention are suitable for the prevention of a pigmentary disorder. As it is, for example known, that some persons have a genetic predisposition for vitiligo, such persons may apply compounds useable according to the invention to prevent vitiligo. Similarly, lentigo often occurs in senile skin and ageing people, and therefore compounds useable according to the invention may be applied early in life in order to prevent pigmentary disorders occuring in senile skin.

### Pharmaceutical compositions

### Pharmaceutically acceptable salts

Examples of pharmaceutically acceptable addition salts include, without limitation, the nontoxic inorganic and organic acid addition salts such as the acetate derived from acetic acid, the aconate derived from aconitic acid, the ascorbate derived from ascorbic acid, the benzenesulfonate derived from benzensulfonic acid, the benzoate derived from benzoic acid, the cinnamate derived from cinnamic acid, the citrate derived from citric acid, the embonate derived from embonic acid, the enantate derived from enanthic acid, the formate derived from formic acid, the fumarate derived from fumaric acid, the glutamate derived from glutamic acid, the glycolate derived from glycolic acid, the hydrochloride derived from hydrochloric acid, the hydrobromide derived from hydrobromic acid, the lactate derived from lactic acid, the maleate derived from maleic acid, the malonate derived from malonic acid, the mandelate derived from mandelic acid, the methanesulfonate derived from methane sulphonic acid, the naphthalene-2-sulphonate derived from naphtalene-2-sulphonic acid, the nitrate derived from nitric acid, the perchlorate derived from perchloric acid, the phosphate derived from phosphoric acid, the phthalate derived from phthalic acid, the salicylate derived from salicylic acid, the sorbate derived from sorbic acid, the stearate derived from stearic acid, the succinate derived from succinic acid, the sulphate derived from sulphuric acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Metal salts of a chemical compound of the invention includes alkali metal salts, such as the sodium salt of a chemical compound of the invention containing a carboxy group.

The chemical compounds of the invention may be provided in unsolved or solvated forms together with a pharmaceutically acceptable solvents such as water, ethanol, and the like. Solvated forms may also include hydrated forms such as the monohydrate, the dihydrate, the hemihydrate, the trihydrate, the tetrahydrate, and the like. In general, solvated forms are considered equivalent to unsolved forms for the purposes of this invention.

### Administration and Formulation

The production of medicaments containing compounds according to the invention and their application can be performed according to well-known pharmaceutical methods.
While a compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides medicaments comprising a compound useable according to the invention, or a pharmaceutically acceptable salt or derivative thereof, together with one or more pharmaceutically acceptable carriers therefor, and, optionally, other therapeutic and/or prophylactic ingredients. The carrier(s) must be "acceptable" in the sense of being compatible with the other ingredients of the formulation and not harmful to the recipient thereof.

A medicament of the invention may be those suitable for oral, rectal, bronchial, nasal, topical, buccal, sub-lingual, transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The compounds useable according to the invention, together with a conventional adjuvant, carrier, or diluent, may thus be placed into the form of medicament and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such Medicament and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The compound useable according to the invention can be administered in a wide variety of oral and parenteral dosage forms. It will be obvious to those skilled in the art that the following dosage forms may comprise, as the active component, either a compounds useable according to the invention or a pharmaceutically acceptable salt of a compounds useable according to the invention.

For preparing a medicament from a compound useable according to the invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.
In powders, the carrier is a finely divided solid which is in a mixture with the finely divided active component. In tablets, the active component is mixed with the carrier having the necessary binding capacity in suitable proportions and compacted in the shape and size desired. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify., Compositions suitable for vaginal administration may be presented as pessaries, tampons, creams, gels, pastes, foams or sprays containing in addition to the active ingredient such carriers as are known in the art to be appropriate. Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired. Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like.

In one embodiment of the present invention, the medicament is applied topically or systemically or via a combination of the two routes.

In a preferred embodiment of the present invention the medicament is applied topically. This reduces possible side effects and limits the necessary treatment to those areas affected. Preferably the medicament is prepared in form of an ointment, a gel, a plaster, an emulsion, a lotion, a foam, a cream of a mixed phase or amphiphilic emulsion system (oil/water-water/oil mixed phase), a liposome, a transfersome, a paste oder a powder.

Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form. Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co. Easton, Pa.).

### Definitions

The linear or branched alkyl having 1 to 6 carbon atoms at R², R³, R⁴ and R⁵ is methyl, ethyl, propyl, isopropyl, butyl, isobutyl, sec-butyl, tert-butyl, pentyl, hexyl and the like.

Aryl at R³ and R⁴ is phenyl, naphthyl and the like.

An aralkyl at R³ and R⁴ is an aralkyl wherein the alkyl moiety is an alkyl having 1 to 4 carbon atoms, and is exemplified by phenylalkyl such as benzyl, 1-phenylethyl, 2-phenylethyl, 3-phenylpropyl, 4-phenylbutyl or the like.

An alkylene having 4 or less carbon atoms, which is formed by R² and R³ directly bonded to each other, is methylene, ethylene, trimethylene, propylene, tetramethylene or the like.

An alkyl having 1 to 10 carbon atoms, which is a substituent at the alkylene having 4 or less carbon atoms formed by R² and R³ directly bonded to each other, is a linear or branched alkyl having 1 to 10 carbon atoms. Examples thereof include methyl, ethyl, propyl, isopropyl, butyl, isobutyl, sec-butyl, tert-butyl, pentyl, hexyl, heptyl, octyl, nonyl, decyl and the like.

An alkyl having 1 to 6 carbon atoms which is a substituent at the ethylene and trimethylene formed by R² and R³ directly bonded to each other, is a linear or branched alkyl having 1 to 6 carbon atoms, which is the same as defined for R², above.

The heterocycle formed by R³ and R⁴ directly or via oxygen atom bonded together with the adjacent nitrogen atom is pyrrolidino, piperidino, morpholino, homopiperidino, homomorpholino or the like.

An alkylene having 2 to 4 carbon atoms formed by R⁶ and R⁷ directly bonded to each other, is ethylene, trimethylene, propylene, tetramethylene or the like.

An alkylene having 2 to 6 carbon atoms at A is ethylene, trimethylene, propylene, tetramethylene, pentamethylene, hexamethylene or the like.

An alkyl having 1 to 6 carbon atoms and an alkyl having 1 to 10 carbon atoms, which are the substituents of alkylene having 2 to 6 carbon atoms at A, are as defined for R², above.

An aryl and aralkyl, which are the substituents of alkylene having 2 to 6 carbon atoms at A, are as defined for R³, above.

### Assays for testing subject compounds

In general, tests for measuring the effect on pigmentary disorders are described in the prior art. For example, an in vitro model consisting of keratinocytes and melanocytes may be used. E.g. the commercially available MelanoDerm Skin Model (MatTek Corporation, Ashland, Massachusetts) uses NHEK (normal human derived keratinocytes)-NHEM (normal human derived melanocytes) cocultures (MelanoDerm Skin model) to model the human epidermis taking into account that melanocytes are dependent on keratinocyte signalling. NHEKs are cultivated in culture inserts placed on the NHEM monolayers according to the instructions of the manufacturer (MatTek Corporation, Ashland, Massachusetts). NHEMs undergo spontaneously melanogenesis up to 3 weeks of culturing. Regarding evaluation of pharmaceutical agents to stimulate skin pigmentation, the tissues darken faster than untreated controls.

Other in vitro assays for determining the effect of a subject compound on pigmentary disorders are in vitro assays based on melanin content analysis.

Melanin content analysis assays are also described in the prior art.

For example, treated and control melanocytes are harvested by trypsinization. Ten percent of the melanocytes are removed and counted on a Coulter counter. The remaining melanocytes are centrifuged and the resulting cell pellet is washed in phosphate-buffered saline pH 7.4 and recentrifuged. The cell pellet is then dissolved in 1ml of 1 M sodium hydroxide. The optical density of this solution is measured spectrophotometrically at 405 nm and is compared to synthetic melanin to determine melanin content per cell. A higher melanin content reflects enhanced pigmentation.

Similarly, the assay can be performed as follows: treated and control melanocytes are harvested by trypsinization, washed with phosphate-buffered saline (PBS), suspended in 500 µl PBS, and counted. The cells are then solubilized by adding 500 µl of 10N NaOH and incubating for 30 min at room temperature. Absorbance at 475 nm is measured and compared with a standard curve for synthetic melanin.

Alternatively, the ¹⁴C-tyrosine melanin-formation assay can be used, which measures the radioactive melanin formed when ¹⁴C-tyrosine is converted to the acid-insoluble melanin biopolymer. For this assay, cell extracts from treated and control cells are incubated with ¹⁴C-tyrosine for 1 hour and absorbed on filter paper. The filter paper is dried then washed in 0.1 mol/L hydrochloric acid three times and placed in a scintillation vial with 5 ml scintillator solution. The radioactivity is counted in triplicate using the same sintillator counter.

Alternatively, the effect of a compound on a pigmentary disorder can be determined by measuring tyrosinase activity, which are well described in the prior art.

For example, tyrosinase activity of cell culture can be determined: treated and control melanocytes are incubated with 1.0 µCi [³H]tyrosine per ml. 700 µl of 10% trichloroacetic acid containing 20% charcoal (charcoal solution) is added to the medium (700 µl), and the mixture is mixed in a vortex for 30 s and then centrifuged at 10,000 rpm for 10 min. Seven hundred microliters of the supernatant is transferred into a new tube and treated twice with the charcoal solution. The radioactivity of the final supernatant is determined in a liquid scintillation counter.

Alternatively, tyrosinase activity can be determined in cell extracts. For example, treated and control melanocytes are collected and washed twice with PBS, and then lyzed in 0.1 M sodium phosphate buffer (pH 6.8) containing 1% Triton X-100. After determining the protein content in the cell extract, 10 µg of each extract is incubated in 100 µl of 100 mM sodium phosphate buffer (pH 6.8) containing 1.0 µCi [³H]tyrosine per ml, 5 µg L-dihydroxyphenylalanine, and 1% Triton X-100 for 15 min at 37°C. After adding 900 µl of charcoal solution, the samples stand for 20 min at 4°C and then are centrifuged at 10,000 rpm for 10 min. The supernatants are applied to a 0.2 ml Dowex-50 column equilibrated in 10% trichloroacetic acid and washed with 0.5 ml of 10% trichloroacetic acid, after which the radioactivity of the effluents is determined in the liquid scintillation counter.

Also, some animal models of varying predictibility and suitability exist. An evaluation of suitability of various vitiligo animal models is described in Vitiligo (eds. S. Hann and J. J.Nordlund, Blackwell Science, Chapter 33: Animal models by L. Lamoreux and R. E. Boissy)

For example, the Smyth chicken exhibiting a genetically inherited form of vitiligo-like depigmentation resulting from the loss of melanocytes in feathers is discussed as vitiligo model. The pigmentation phenotype of this animal model appears to involve an immune response. Also, the so-called vitiligo mouse is described. In this model no immune component is involved and the disorder is not polyfactorial; i.e. unlike in humans.

Vitiligo, for example, is not a simple, one-locus-disease, and inheritance is not its only cause. Therefore, in the case of vitiligo, animal models must be used with the knowledge that the disorder observed in the genetically defective animal will not be homologous with the disorder of the average human vitiligo patient. Thus, the study of one animal model, especially if the defect is caused by one gene locus in the model, need not be more predictive than an in vitro model.

None of the aforementioned tests has proved particular useful on its own when it came to identifying compounds useful in the treatment and/or prevention of pigmentary disorders, given that not many compounds are known to have a pronounced positive effect on the onset of pigmentary disorders (i.e. compounds which induce the onset of pigmentary disorders), or to have a negative effect on the onset of pigmentary disorders (i.e. compounds which inhibit or prevent the onset of pigmentary disorders). This shortcoming of the prior art has now been overcome, in that the present inventors have found that surprisingly the isoquinoline-derivatives according to the present invention, as defined before, provide for compounds which have either an inhibiting or inducing effect on the onset of pigmentary disorders. Therefore, according to another aspect of the present invention, it provides for the use of a compound according to formula (I), as defined above or as defined in specific embodiments thereafter, in a screening assay for compounds that induce or inhibit the onset of pigmentary disorders. In one embodiment, such screening assay is an in-vitro-model consisting of a co-culture of keratinocytes and melanocytes as described above. In another embodiment the screening assay is an in-vitro-assay based on melanin content analysis, as described above. In another embodiment, the screening assay is based on the measurement of tyrosinase activity, as described above. In yet another embodiment, the screening assay is based on an animal model, as described above.

In all these screening assays the isoquinoline-compounds according to the present invention serve as positive or negative controls in that they inhibit/prevent or induce the onset of pigmentary disorders, and the response of the assay system towards these controls is compared with the response of the assay system towards a subject compound to be tested.

Further aspects of the present invention will become evident by the following example which is given to merely illustrate the invention, not to limit the same.

### Example 1: Effect of isquinoline-derivatives on melanocyte function

A possibility to test the effect of a compound on melanocyte function as a measure for pigmentation, the melanocyte dendrite outgrowth assay can be performed. Vitiligo melanocytes for example have only stubby dendrites. Increasing dendricity as a prerequisite for the transfer of the melanosomes from melanocytes to the surrounding keratinocytes rescues this defect.

For this assay, normal human epidermal melanocytes are obtained from Clonetics (neonatal melanocytes) or BIOalternatives (melanocytes from adults) and are cultured according to the manufacturers' protocol. In short, after thawing the melanocytes are cultured in T-75 flasks in melanocyte culture media (provided by the manufacturer) until they reach 70-80% confluency. The melanocytes are then trypsinized, transferred into 5-10 new T-75 flasks, and grown again to same confluency as described above. After this expansion step the melanocytes are immediately used for the Melanocyte Dendrite Outgrowth Assay or are frozen for later use.

For the assay, the melanocytes are seeded on glass coverslips. After 24 hours, the melanocytes are treated with Forskolin (Sigma-Aldrich), α-melanocyte stimulating hormone (α-MSH; Sigma-Aldrich), keratinocyte conditioned media (KCM; see below) or different concentrations of 1-(5-isoquinolinesulfonyl)homopiperazine or another subject compound to be tested, for 24, 48 or 72 hours. Forskolin, α-MSH, and KCM (Keratinocyte conditioned media) are known to cause increased melanocyte dendricity and are used as positive controls. Cells treated with media only are used as a negative control. Melanocyte dendrite outgrowth is analyzed by immunofluorescence microscopy. To prepare the cells for microscopy they are briefly rinsed with PBS and fixed with methanol (at -20°C) for 2 min. Subsequently, the melanocytes are permeabilized with 0.1 % Triton-X 100 for 2 min. The primary anti-human tyrosinase antibody diluted in PBS/1% BSA is added to the cells for 30-60 min. The cells are washed for 10 min in PBS and exposed to the secondary antibody. The coverslips are rinsed in PBS for 10 min and are observed and photographed with a Zeiss Axioskop 2 fluorescence microscope. Cells are visualized using phase contrast microscopy or fluorescence microscopy.

An increased dendricity proves suitability for treating hypopigmentary disorders, especially vitiligo. A reduced dendricity proves suitability for treating hyperpigmentary disorders, especially lentigo and ephelides.

The features of the present invention disclosed in the specification, the claims and/or in the accompanying drawings, may, both separately, and in any combination thereof, be material for realising the invention in various forms thereof.

## Claims

1. Use of a compound according to formula (I): 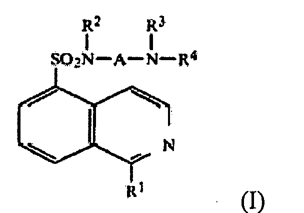 or an isomer thereof, or a pharmaceutically acceptable salt thereof, for the manufacture of a medicament for treating and/or preventing pigmentary disorders
wherein
R¹ is a hydrogen, a chlorine or a hydroxy, and
when R¹ is a hydrogen:
A is an alkylene having 2 to 6 carbon atoms, which optionally has an alkyl having 1 to 10 carbon atoms, aryl or aralkyl as a substituent;
R² is a hydrogen or a linear or branched alkyl having 1 to 6 carbon atoms, an aryl or an aralkyl;
R³ is a hydrogen, or a linear or branched alkyl having 1 to 6 carbon atoms, an aryl or an aralkyl;
R⁴ is a hydrogen, a linear or branched alkyl having 1 to 6 carbon atoms, an aryl or an aralkyl, or a benzoyl, a cinnamyl, a cinnamoyl, a furoyl or a group of the following formula 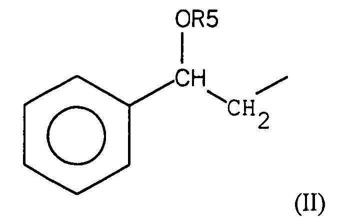 wherein R⁵ is a linear or branched alkyl having 1 to 6 carbon atoms or a group of the following formula 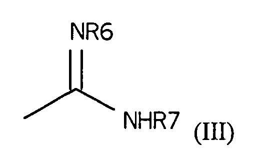 wherein R⁶ and R⁷ are hydrogen or directly bonded to form alkylene having 2 to 4 carbon atoms; or
R² and R³ are directly bonded to form an alkylene having 4 or less carbon atoms, which is optionally substituted by an alkyl having 1 to 10 carbon atoms, phenyl or benzyl, or
R³ and R⁴ directly or in combination via oxygen atom form a heterocycle together with the adjacent nitrogen atom, and
when R¹ is a chlorine or a hydroxy:
A is an alkylene having 2 to 6 carbon atoms, which is optionally substituted by an alkyl having 1 to 6 carbon atoms,
R² and R³ are each independently hydrogen, a linear or branched alkyl having 1 to 6 carbon atoms, or R₂ and R₃ are directly bonded to each other to form ethylene or trimethylene, which ethylene or trimethylene is optionally substituted by an alkyl having 1 to 6 carbon atoms; and
R⁴ is hydrogen, a linear or branched alkyl having 1 to 6 carbon atoms or an amidino.

2. Use according to claim 1, **characterized in that** the compound is selected from
1-(5-isoquinolinesulfonyl)homopiperazine
1-(5-isoquinolinesulfonyl)-2-methylhomopiperazine
1-(5-isoquinolinesulfonyl)-3-methylhomopiperazine
1-(5-isoquinolinesulfonyl)-6-methylhomopiperazine
1-(5-isoquinolinesulfonyl)-2,3-dimethylhomopiperazine
1-(5-isoquinolinesulfonyl)-3,3-dimethylhomopiperazine
1-(5-isoquinolinesulfonyl)-3-ethylhomopiperazine
1-(5-isoquinolinesulfonyl)-3-propylhomopiperazine
1-(5-isoquinolinesulfonyl)-3-isobutylhomopiperazine
1-(5-isoquinolinesulfonyl)-3-phenylhomopiperazine
1-(5-isoquinolinesulfonyl)-3-benzylhomopiperazine
1-(5-isoquinolinesulfonyl)-6-ethylhomopiperazine
1-(5-isoquinolinesulfonyl)-6-propylhomopiperazine
1-(5-isoquinolinesulfonyl)-6-butylhomopiperazine
1-(5-isoquinolinesulfonyl)-6-pentylhomopiperazine
1-(5-isoquinolinesulfonyl)-6-hexylhomopiperazine
1-(5-isoquinolinesulfonyl)-6-phenylhomopiperazine
1-(5-isoquinolinesulfonyl)-6-benzylhomopiperazine
1-(5-isoquinolinesulfonyl)-4-methylhomopiperazine
1-(5-isoquinolinesulfonyl)-4-ethylhomopiperazine
1-(5-isoquinolinesulfonyl)-4-propylhomopiperazine
1-(5-isoquinolinesulfonyl)-4-butylhomopiperazine
1-(5-isoquinolinesulfonyl)-4-hexylhomopiperazine
N-(2-aminoethyl)-1-chloro-5-isoquinolinesulfonamide
N-(4-aminoethyl)-1-chloro-5-isoquinolinesulfonamide
N-(2-amino-1-methylethyl)-1-chloro-5-isoquinolinesulfonamide
N-(2-amino-1-methylpentyl)-1-chloro-5-isoquinoline
N-(3-amino-2-methylbutyl)- 1-chloro-5-isoquinolinesulfonamide
N-(3-di-n-butylaminopropyl)-1-chloro-5-isoquinolinesulfonamide
N-( N-cyclohexyl-N-methylaminoethyl)-1-chloro-5-isoquinolinesulfonamide
N-(2-guanidinoethyl)-1-chloro-5-isoquinolinesulfonamide
N-(2-guanidinobutyl)-5-chloro-5-isoquinolinesulfonamide
N-(2-guanidino-1-methylethyl)-1-chloro-5-isoquinolinesulfonamide
N-(2-guanidinomethylpentyl)-1-chloro-5-isoquinolinesulfonamide
N-(2-guanidino-3-methylbutyl)-1-chloro-5-isoquinolinesulfonamide
N-(3-guanidino-2-methylpropyl)-1-chloro-5-isoquinolinesulfonamide
N-(4-guanidino-3-methylbutyl)-1-chloro-5-isoquinolinesulfonamide
2-methyl-4-( 1-chloro-5-isoquinolinesulfonyl)piperazine
2-ethyl-4-(1-chloro-5-isoquinolinesulfonyl)piperazine
2-isobutyl-4-(1-chloro-5-isoquinolinesulfonyl)piperazine
2,5-dimethyl-4-(1-chloro-5-isoquinolinesulfonyl)piperazine
1-methyl-4-(1-chloro-5-isoquinolinesulfonyl)piperazine
1-amidino-4-(1-chloro-5-isoquinolinesulfonyl)piperazine
1-amidino4-(1-chloro-5-isoquinolinesulfonyl)homopiperazine
1-amidino-3-methyl-4-(1-chloro-5-isoquinolinesulfonyl)piperazine
1-amidino-2,5-dimethyl-4-(1-chloro-5-isoquinolinesulfonyl)piperazine
N-(2-aminoethyl)-1-hydroxy-5-isoquinolinesulfonamide
N-(4-aminobutyl)-1-hydroxy-5-isoquinolinesulfonamide
N-(2-amino-1-methylethyl)-1-hydroxy-5-isoquinolinesulfonamide
N-(2-amino-1-methylheptyl)-1-hydroxy-5-isoquinolinesulfonamide
N-(3-amino-2-methylbutyl)-1-hydroxy-5-isoquinolinesulfonamide
N-[3-(N,N-dibutylamino)propyl]-1-hydroxy-5-isoquinolinesulfonamide
N-[2-(N-cyclohexyl-N-methylamino)ethyl]-1-hydroxy-5-isoquinolinesulfonamide
N-(2-guanidinoethyl)-1-hydroxy-5-isoquinolinesulfonamide
N-(4-guanidinobutyl)-1-hydroxy-5-isoquinolinesulfonamide
N-(2-guanidino-1)-methylethyl)-1-hydroxy-5-isoquinolinesulfonamide
N-(1-guanidinomethylpentyl)-1-hydroxy-5-isoquinolinesulfonamide
N-(2-guanidino-3-methylbutyl)-1-hydroxy-5-isoquinolinesulfonamide
N-(3-guanidino-2-methylpropyl)-1-hydroxy-5-isoquinolinesulfonamide
N-(4-guanidino-3-methylbutyl)-1-hydroxy-5-isoquinolinesulfonamide
2-methyl-4-( 1-hydroxy-5-isoquinolinesulfonyl)piperazine
2-ethyl-4-(1-hydroxy-5-isoquinolinesulfonyl)piperazine
2-isobutyl-4-(1-hydroxy-5-isoquinolinesulfonyl)piperazine
2,5-dimethyl-4-( 1-hydroxy-5-isoquinolinesulfonyl)piperazine
1-methyl-4-(1-hydroxy-5-isoquinolinesulfonyl)piperazine
1-amidino-4-(1-hydroxy-5-isoquinolinesulfonyl)piperazine
1-amidino-4-(1-hydroxy-5-isoquinolinesulfonyl)homopiperazine
1-amidino-3-methyl-4-(1-hydroxy-5-isoquinolinesulfonyl)piperazine
1-amidino-2,5-dimethyl-4-(1-hydroxy-5-isoquinolinesulfonyl)piperazine
N-(2-methylaminoethyl)-1-chloro-5-isoquinolinesulfonamide
N-(2-ethylaminoethyl)-1-chloro-5-isoquinolinesulfonamide
N-(2-propylaminoethyl)-1-chloro-5-isoquinolinesulfonamide
N-(2-butylaminoethyl)-1-chloro-5-isoquinolinesulfonamide
N-(2-hexylaminoethyl)-1-chloro-5-isoquinolinesulfonamide
1-(1-chloro-5-isoquinolinesulfonyl)piperazine
1-(1-chloro-5-isoquinolinesulfonyl)homopiperazine
N-(2-methylaminoethyl)-1-hydroxy-5-isoquinolinesulfonamide
N-(2-ethylaminoethyl)-1-hydroxy-5-isoquinolinesulfonamide
N-(2-propylaminoethyl)-1-hydroxy-5-isoquinolinesulfonamide
N-(2-butylaminoethyl)-1-hydroxy-5-isoquinolinesulfonamide
N-(2-hexylaminoethyl)-1-hydroxy-5-isoquinolinesulfonamide
1-(1-hydroxy-5-isoquinolinesulfonyl)piperazine
1-(1-hydroxy-5-isoquinolinesulfonyl)homopiperazine
1-(5-isoquinolinesulfonyl)-4-methylpiperazine
1-(5-isoquinolinesulfonyl)-4-n-hexylpiperazine
1-(5-isoquinolinesulfonyl)-4-cinnamylpiperazine
1-(5-isoquinolinesulfonyl)piperazine
N-(4-aminobutyl)-5-isoquinolinesulfonamide
N-(3-di-n-butylaminopropyl)-5-isoquinolinesulfonamide
1-(5-isoquinolinesulfonyl)-3-methylpiperazine
1-(5-isoquinolinesulfonyl)-3-isobutylpiperazine
1-(5-isoquinolinesulfonyl)-2,5-dimethylpiperazine
N-(3-guanidino-2-phenylpropyl)-5-isoquinolinesulfonamide
N-(6-guanidino-1-methylheptyl)-5-isoquinolinesulfonamide
2-[2-(5-isoquinolinesulfonamide)ethylamino]-2-imidazoline
2-amidino-1-(5-isoquinolinesulfonyl)piperazine
4-amidino-2,5-dimethyl-1-(5-isoquinolinesulfonyl)piperazine
4-amidino-1-(5-isoquinolinesulfonyl)homopiperazine
4-(N1,N2-dimethylamidino)-1-(5-isoquinolinesulfonyl)piperazine
4-amidino-3-butyl-1-(5-isoquinolinesulfonyl)piperazine
4-hexyl-1-(5-isoquinolinesulfonyl)ethylenediamine
N-(4-guanidinobutyl)-5-isoquinolinesulfonamide
N-(2-guanidinoethyl)-5-isoquinolinesulfonamide

3. Use according to claim 2, **characterized in that** the compound is 1-(5-isoquinolinesulfonyl)homopiperazine.

4. Use according to claims 1 to 3, **characterized in that** the pigmentary disorder is a pigmentary disorder of skin or hair, preferably of the skin.

5. Use according to claims 1 to 4, **characterized in that** the pigmentary disorder is a hypopigmentary disorder.

6. Use according to claim 5, **characterized in that** the pigmentary disorder is selected from albinism, vitiligo, piebaldism, Pityariasis alba, Hypomelanoses, Leukodermas, hypopigmentation occurring e.g. after externally induced peels, e.g. chemical peels, e. g. with phenol, or laser or cryo-surgery of the skin, Chediak-Higashi syndrome, Hermansky-Pudlak syndrome, the Angelman and Prader-Willi syndrome.

7. Use according to claim 6 **characterized in that** the pigmentary disorder is vitiligo.

8. Use according to claims 1 to 4, **characterized in that** the pigmentary disorder is a partial hyperpigmentation disorder, preferably selected from Lentigo and ephelides

9. Use according to any of the foregoing claims, **characterized in that** the medicament is applied topically or systemically or via a combination of the two routes.

10. Use according to any of the foregoing claims 1 to 4, **characterized in that** the medicament is prepared in form of an ointment, a gel, a plaster, an emulsion, a lotion, a foam, a cream of a mixed phase or amphiphilic emulsion system (oil/water-water/oil mixed phase), a liposome, a transfersome, a paste or a powder, or a solution or suspension.
